# EUROPÄISCHE PATENTSCHRIFT

(11) **EP 1 028 103 B1**
(45) Veröffentlichungstag und Bekanntmachung des Hinweises auf die Patenterteilung: **04.09.2002**
(21) Anmeldenummer: 00101759.9
(22) Anmeldetag: 28.01.2000
(51) Int. Cl.: C07C 67/00, C07C 69/017, C07C 69/16

(54) **Verfahren zur Herstellung von 2,3,5-Trimethylhydrochinondiestern**
Process for the preparation of 2,3,5-trimethylhydroquinone diester
Procédé pour la préparation de diesters de 2,3,5-trimethylhydroquinone

(30) Priorität: 11.02.1999 DE 19905685
(43) Veröffentlichungstag der Anmeldung: 16.08.2000
(73) Patentinhaber: Degussa AG, 40474 Düsseldorf (DE)
(72) Erfinder: Krill, Steffen, Dr., 67346 Speyer (DE); Huthmacher, Klaus, Dr., 63584 Gelnhausen (DE)

(56) Entgegenhaltungen:
- EP-A- 0 850 910
- EP-A- 0 850 912
- DE-A- 2 149 159

## Beschreibung

Die Erfindung betrifft ein verbessertes Verfahren zur Herstellung von 2,3,5-Trimethylhydrochinondiestern durch Umlagerung von 2,6,6-Trimethylcyclohex-2-en-1,4-dion (4-Oxo-Isophoron, Ketoisophoron) in Gegenwart eines gelösten, sauren Katalysators und eines Acylierungsmittels, wie zum Beispiel Carbonsäureanhydriden oder Carbonsäurehalogeniden. Der 2,3,5-Trimethylhydrochinondiester kann gegebenenfalls anschließend zum freien 2,3,5-Trimethylhydrochinon (TMHQ) verseift werden, das ein wertvoller Baustein bei der Synthese von Vitamin E ist.

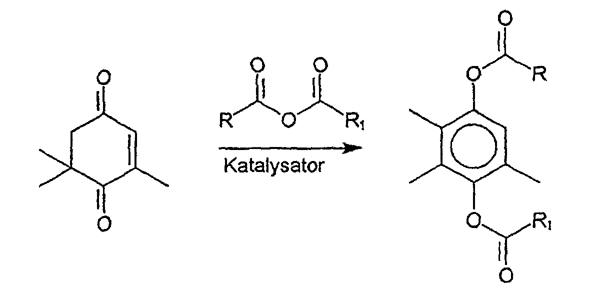

### Stand der Technik

2,3,5-Trimethylhydrochinondiester und das entsprechende TMHQ sind wichtige Zwischenprodukte, die bei der Produktion von Vitamin-E bzw. Vitamin-E-Acetat verwendet werden. Neben den bekannten Herstellungsverfahren aus aromatischen Ausgangsmaterialien kann 2,3,5-Trimethylhydrochinon aus einer nichtaromatischen Verbindung, dem 2,6,6-Trimethylcyclohex-2-en-1,4-dion, durch Umlagerung unter acylierenden Bedingungen und anschließender Hydrolyse hergestellt werden.

In der Patentschrift DE 26 46 172 C2 wird ein Verfahren beschrieben, bei dem 2,6,6-Trimethylcyclohex-2-en-1,4-dion in der Dampfphase bei hoher Temperatur in Kontakt mit einem sauren Katalysator direkt zu Trimethylhydrochinon umgelagert wird. Allerdings ist die Ausbeute bei diesem Verfahren nur gering (50 % bei 30 % Umsatz). Wird die Aromatisierung von 2,6,6-Trimethylcyclohex-2-en-1,4-dion in Gegenwart eines Acylierungsmittels durchgeführt, so werden Trimethylhydrochinondiester erhalten, die durch anschließende Hydrolyse zum Trimethylhydrochinon führen.

So wird beispielsweise gemäß Bull. Korean. Chem. Soc. 1991, 12, 253 die Umlagerung in 5 %-iger Lösung in Acetanhydrid durch Zugabe von fünf Äquivalenten konzentrierter Schwefelsäure durchgeführt. Der Trimethylhydrochinondiester wird dabei lediglich mit 30 % Ausbeute erhalten.

In einem weiteren Verfahren gemäß DE-OS 2 149 159 kann 2,6,6-Trimethylcyclohex-2-en-1,4-dion in Gegenwart von Acetanhydrid in einer durch Protonen- oder Lewissäurenkatalysierten Umlagerung zu Trimethylhydrochinondiacetat umgewandelt werden, welches anschließend zu Trimethylhydrochinon verseift wird.

Obwohl Ausbeuten und Umsätze von Ketoisophoron nach dieser Verfahrensweise mäßig bis gut sind (maximal 66 % TMHQ-Ausbeute, bezogen auf eingesetzes Ketoisophoron), werden große Mengen an starken Säuren (bis 150 mol% bezogen auf Ketoisophoron) und große Überschüsse an Acetanhydrid (5 - 10 Mol Ac₂O/1 Mol Ketoisophoron) verwendet, was das Verfahren aus technischer Sicht wenig attraktiv macht.

Nach einem neueren Verfahren (DE-OS 196 27 977) wird Ketoisphoron in Gegenwart eines nur doppelt stöchiometrischen Acetanhydridäquivalents mit homogen gelosten Supersäuren (H₀ < -11,9) als Katalysatoren in flüssiger Phase umgesetzt. Besonders hohe Selektivitäten werden mit Trifluormethansulfonsäure, Chlorsulfonsäure und Oleum verschiedener SO₃-Konzentrationen erreicht. Nachteilig an diesem Verfahren ist die Verwendung der beschriebenen Katalysatoren, deren Korrosivität erhebliche Werkstoffprobleme mit sich bringt. Die Verwendung von Trifluormethansulfonsäure als Katalysator ist teuer und aufwendig, die Handhabung kompliziert und eine Recyclierung des Reagenzes nur bedingt möglich.

### Aufgabe

Die Aufgabe der Erfindung besteht darin, ein verbessertes Verfahren zur Herstellung von 2,6,6-Trimethylcyclohex-2-en-1,4-dion-diestern zu finden, das insbesondere unter Verwendung eines leicht handhabbaren, wirtschaftlichen Katalysators abläuft. Aus den Estern sind gegebenenfalls die entsprechenden Hydrochinone durch Hydrolyse zu gewinnen.

### Lösung der Aufgabe

Gegenstand der Erfindung ist ein Verfahren zur Herstellung von Trimethylhydrochinondiestern,

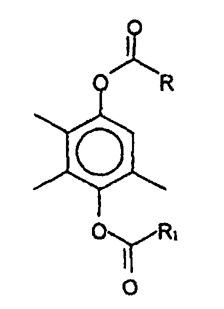

R, R1 gleich oder verschieden: ein gegebenenfalls substituierter aliphatischer, alicyclischer C₁ bis C₂₀-Rest, insbesondere einen aliphatischer C₂ bis C₄-Rest, oder Aryl, insbesondere Phenylen, durch Umsetzung von 2,6,6-Trimethyl-cyclohex-2-en-1,4-dion(Ketoisophoron oder KIP) 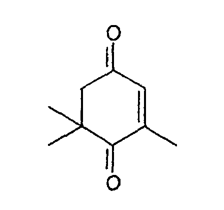 mit einem Acylierungsmittel in Gegenwart katalytischer Mengen einer Protonensäure, das dadurch gekennzeichnet ist, daß man ortho-Borsäure und/oder Borsäureanhydrid einerseits und eine oder mehrere Carbonsäure(n), ausgewählt aus der Gruppe der Hydroxycarbonsäuren, der Di-oder Tricarbonsäuren, die gegebenenfalls auch Hydroxygruppen enthalten, andererseits einsetzt.

Dieses kombinierte Katalysatorsystem ist wirtschaftlich außerordentlich günstig und zusätzlich leicht handhabbar.

Gleichzeitig sind Ausbeuten und Selektivitäten mit den aus dem Stand der Technik bekannten als gleichwertig einzustufen.

Die Wirkung des Katalysatorsystems beruht auf einer sich aus der Bor-haltigen Verbindung einerseits und den eingesetzten Carbonsäuren andererseits in situ bildenden Katalysatorspezies, deren pKₛ-Wert niedriger ist als der pKₛ-Wert von Borsäure.

Diese Katalysatorkombination wird bevorzugt in einer Menge von 0,1 bis 10 mol%, bezogen auf das eingesetzte Ketoisophoron, verwendet. Sie liegt gelöst in dem Reaktionsgemisch vor.

Als Borsäurederivat können verschiedenste Borverbindungen eingesetzt werden, insbesondere Borsäuretriester, Boroxide und Borsäure, wobei als besonders bevorzugter Katalysator Borsäure eingesetzt wird. Als Cokatalysator können verschiedene oligofunktionelle Verbindungen eingesetzt werden, die mit Borsäurederivaten unter Erhöhung der Borkoordinationssphäre stabile komplexe Borsäuren bilden, deren Säurestärke stärker als die der entsprechenden freien Borsäure ist.

Das Verhältnis von Borkomponente und Cokatalysator kann in Bereichen zwischen 1 : 1 bis 1 : 10 (Molverhältnis) variiert werden, wobei bei bifunktionellen Cokatalysatoren ein Verhältnis von 1 : 2 besonders bevorzugt wird. Die aktive Katalysatorspezies entsteht in situ durch Reaktion des binären Katalysatorsystems aus Borsäurederivat einerseits und dem Cokatalysator andererseits in Gegenwart des Acylierungsmittels.

Als Cokatalysatoren werden insbesondere Hydroxysäuren der allgemeinen Formel

R₂-CO₂H (I)

bezeichnet, in der bedeuten:
- R₂:: Aryl, insbesondere Phenylen, Naphtyl, jeweils durch OH substituiert, HO-(CH₂)ₒ-, CH₃(CHOH)ₙ(CH₂)ₘ- mit m eine ganze Zahl von 0 bis 20, insbesondere 0 bis 8, und
- n:: eine ganze Zahl von 1 bis 5, insbesondere 1 bis 4,
- o:: eine ganze Zahl von 1 bis 6

Zu den besonders geeigneten Hydroxycarbonsäuren zählen Glykolsäure, Milchsäure, Mandelsäure, Weinsäure (unabhängig von der Konfiguration), Zitronensäure, insbesondere Salicylsäure oder Acetylsalicylsäure, aber auch Hydroxygruppen enthaltende Aminosäuren wie Serin oder Threonin und Aldonsäure.

Bevorzugt eingesetzt werden auch Dicarbonsäuren der allgemeinen Formel

HO₂C-R₃-CO₂H (II),

in der bedeuten:
- R₃:: Aryl, insbesondere Phenylen, Naphtyl, (CH₂)ₘ, wobei ₘ dieselbe Bedeutung wie oben hat, oder (CH₂)ₘ(CHX)ₒ, wobei ₒ einer ganzen Zahl von 1 bis 5 entspricht, mit X: OH,H oder (CH₂)_{P}-(CX) (COOH)- (CH₂)_{P} mit _{P} = 1 bis 3 oder Alkenyl mit C₂ bis C₆.

Besonders geeignet sind Oxalsäure, Malonsäure, Apfelsäure, Bernsteinsäure, Glutarsäure, Adipinsäure, Pimelinsäure, Korksäure, Azelainsäure, Sebacinsäure, insbesondere Oxalsäure, sowie aromatische Dicarbonsäuren wie Phtalsäure, Isophtalsäure und Terephtalsäure und 2,6-Naphtalincarbonsäure oder Tricarbonsäuren wie Trimesinsäure oder die Zitronensäure, sowie ungesättigte Dicarbonsäuren wie Fumarsäure und Maleinsäure, aber auch Polyhydroxydicarbonsäuren.

Man setzt als Acylierungsmittel bevorzugt Verbindungen der allgemeinen Formel ein, in der bedeuten:

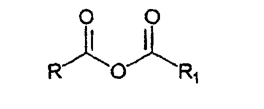

R, R₁, gleich oder verschieden: einen gegebenenfalls substituierten aliphatischen, alicyclischen C₁ bis C₂₀-Rest, insbesondere einen aliphatischen C₂ bis C₄-Rest, Aryl, insbesondere Phenylen.

Besonders geeignet ist das Anhydrid der Essigsäure.

Weitere geeignete Anhydride sind die der Propionsäure, Buttersäure, Isobuttersäure, Cyclohexancarbonsäure oder Benzoesäure.

Einsetzbar sind ebenso die Anhydride der Chloressigsäure, Trihalogenessigsäure oder Trifluormethansulfonsäure, auch wenn sie nicht bevorzugt werden.

Das Verhältnis zwischen Ketoisophoron und Acylierungsmittel kann in weiten Bereichen variiert werden, jedoch ist ein Verhältnis KIP/Acylierungsmittel von 1 : 2 bis 1 : 3 besonders bevorzugt. Auch andere Acylierungsreagenzien wie Carbonsäurehalogenide insbesondere die den genannten Anhydriden ensprechenden Chloride, Enolester und Diketen können als Syntheseäquivalent und Ersatz für das bevorzugt verwendete Acetanhydrid eingesetzt werden.

Das erfindungsgemäße Verfahren kann unter die Verwendung inerter organischer Lösungsmittel durchgeführt werden. Die Konzentration der Reaktanten im Lösungsmittel hat nur einen unwesentlichen Einfluß auf das Produktbild der Umsetzung. Besonders bevorzugt arbeitet man lösungsmittelfrei, sodaß aufwendige Aufarbeitungsoperationen bzw. Recyclierungen von Lösungsmittel vermieden werden.

Erfolgt die Umlagerung in Gegenwart organischer Lösungsmittel, dienen als geeignete Vertreter aliphatische und cyclische Ester, z. B. Essigsäureethylester, Essigsäurepropylester, Essigsäureopropylester und γ-Butyrolacton; Kohlenwasserstoffe, zum Beispiel Hexan, Heptan, Toluol und Xylol; und Ketone, zum Beispiel Isobutylmethylketon, Diethylketon und Isophoron.

Die KIP Umlagerung findet bei Temperaturen zwischen -80 bis +150°C statt, wobei der Temperaturbereich zwischen -30 bis +50°C besonders bevorzugt ist. Bei noch höheren Temperaturen nimmt die Nebenproduktbildung auf Kosten des Trimethylhydrochinonesters zu.

In einer Ausführungsform des erfindungsgemäßen Verfahrens wird der entstandene Trimethylhydrochinondiester direkt aus der bei der Reaktion entstandenen Carbonsäure auskristallisiert. Es ist aber auch möglich, die Isolierung durch Zugabe eines geeigneten Lösungsmittels nach Abdestillieren der freien Carbonsäure durchzuführen.

In einer weiteren Ausführungsform verseift man das entstandene TMHQ-Diacetat ohne Isolierung, indem man zur Rohmischung der Umlagerung Wasser zugibt. Als Verseifungskatalysator kann der gleiche Katalysator genutzt werden, der bereits für die Umlagerung von Ketoisophoron eingesetzt wurde. Die Isolierung des freien Trimethylhydrochinons erfolgt in an sich bekannter Weise durch Kristallisation aus einem geeigneten Medium.

Damit ist es gelungen, die Trimethylhydrochinonsynthese durch die Bereitstellung eines günstigen, einfach zugänglichen, gut handhabbaren Katalysatorsystems auf Basis eines Borsäurederivates und der Durchführung als Kreislaufprozess unter Recycling des Katalysators auf ein technisch durchführbares, wirschaftliches Niveau zu heben.

Bei der erfindungsgemäßen Herstellung von 2,3,5-Trimethylhydrochinondiestern bzw. 2,3,5-Trimethylhydrochinon ergeben sich folgende wesentliche Vorteile gegenüber dem Stand der Technik:

Die Ausbeuten liegen mit einer Ausbeute von bis zu 95 % bezogen auf das eingesetzte Ketoisophoron im Bereich der höchsten Werte, die mit Trifluormethansulfonsäure als Katalysator erzielt werden können.

Der Katalysator hat in der Handhabung im Vergleich zu den aus dem Stand der Technik bekannten Supersäuren wesentliche Vorteile bezüglich Dosierung, Toxizität und Korrosivität. So kann der Katalysator durch einfaches Verrühren der Katalysatorkomponenten vor dem Einsatz hergestellt werden, oder er entsteht in situ durch sukzessive Zugabe der Komponenten zum Carbonsäureanhydrid.

### Beispiele

### Beispiel 1

Zu einer Lösung von 28,3 ml Acetanhydrid (=0,3 Mol) gibt man unter Rühren bei Raumtemperatur 1 g (= 5,32 mmol) eines Katalysatorgemischs aus Borsäure und Oxalsäure in einem molaren Verhältnis von 1 : 2. Geht man davon aus, daß die aktive Katalysatorspezies ein 1 : 2 Gemisch aus den beiden Komponenten unter Austritt von 2 Moläquivalenten Wasser ist, dann hat der aktive Katalysator ein Molgewicht von 187,86 g/Mol. Dies entspricht einer Katalysatormenge von 6,62 wt% bzw. 5,32 Mol% bezogen auf eingesetztes KIP.

Es erfolgt ein rascher Temperaturanstieg bis auf 50°C. Danach werden während 20 min. 15,1 g KIP (= 0,1 Mol) zu dieser Lösung getropft und die Exotherme mittels Wasserbad so abgefangen, daß die Temperatur konstant bei 50°C gehalten wird. Nach einer Stunde Nachreaktionszeit wird auf Eiswasser gegossen, die Kristalle abgesaugt und mit Wasser gewaschen. Die gaschromatographische Analyse zeigt einen quantitativen Umsatz, Ausbeute und Selektivität an Trimethylhydrochinondiacetat betragen 88,2 %.Die Nebenproduktbildung beträgt nach GC ca. 11,6 % Trimethylbrenzcatechindiacetat, das mittels Kristallisation abgetrennt werden kann.

### Beispiel 2

Man legt eine Mischung von 28,3 ml Acetanhydrid (= 0,3 Mol) und 0,4 g einer Katalysatormischung (= 2,12 mmol) aus Borsäure und Oxalsäure (molares Verhältnis 1 : 2) bei 30°C vor. Dies entspricht bezogen auf eingesetztes KIP einer Katalysatormenge von 2,13 Mol% und 2,63 wt%. Bei 30°C wird KIP langsam zugetropft (15,2 g = 0,1 Mol), danach in üblicher Weise aufgearbeitet. Ausbeute und Selektivität der Umsetzung betragen nach GC 90,7 %, die Nebenproduktbildung betragt 8,8 %.

### Beispiel 3

Man arbeitet wie unter Ausführungsbeispiel 1 beschrieben und erniedrigt die Katalysatormenge bezogen auf KIP auf 1,33 mol% (= 0,25 g einer Mischung aus Borsäure und Oxalsäure im Verhältnis 1 : 2) oder 1,64 Gew.%. Der Umsatz bei diesen Bedingungen unter Einhaltung gleicher Reaktionszeiten beträgt 39,7 %, die TMHQ-DA Selektivität beträgt 85,6 %. Nicht umgesetztes KIP kann durch geeignete verfahrenstechnische Operationen wie Destillation oder Extraktion wiedergewonnen und vom Produkt getrennt werden.

### Beispiel 4

Man arbeitet mit 5,32 Mol% einer Katalysatormischung aus Borsäure und Oxalsäure im molaren Verhältnis 1 : 2 und führt die Umsetzung bei 10 bis 15°C durch, indem man die Exotherme, die beim Zutropfen von KIP auftritt, mit einem Eiswasserbad abfängt. Selektivität und Ausbeute an TMHQ-DA betragen nach dieser Verfahrensweise 92,9 %, die Nebenproduktausbeute beträgt 6,9 %.

## Patentansprüche

1. Verfahren zur Herstellung von Trimethylhydrochinondiestern 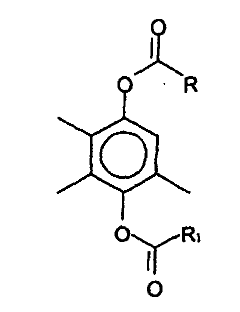 wobei R und R₁ ein gegebenenfalls substituierter aliphatischer, alicyclischer C₁ bis C₂₀-Rest, oder Aryl sind und wobei R und R₁ gleich oder verschieden sind, durch Umsetzung von 2,6,6-Trimethyl-cyclohex-2-en-1,4-dion (Ketoisophoron oder KIP) 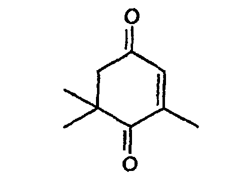 mit einem Acylierungsmittel in Gegenwart katalytischer Mengen einer Protonensäure, **dadurch gekennzeichnet, daß** man als sauren Katalysator ortho-Borsäure und/oder Borsäureanhydrid oder einem Borsäuretriester einerseits und eine oder mehrere Carbonsäure(n), ausgewählt aus der Gruppe der Hydroxycarbonsäuren, der Di- oder Tricarbonsäuren, die gegebenenfalls auch Hydroxygruppen enthalten, andererseits einsetzt.

2. Verfahren gemäß Anspruch 1, **dadurch gekennzeichnet, daß** R und R₁ ein aliphatischer C₂ bis C₄-Rest oder Phenylen sind.

3. Verfahren gemäß Anspruch 1, **dadurch gekennzeichnet, daß** man Dicarbonsäuren der allgemeinen Formel
HO₂C-R₃-CO₂H (II)
in der R₃ Aryl oder (CH₂)ₘ ist , wobei m eine ganze Zahl von 0-20 darstellt, oder (CH₂)ₘ(CHX)ₒ, wobei o einer ganzen Zahl von 1 bis 5 entspricht, mit X: OH,H oder (CH₂)_{P}-(CX) (COOH)-(CH₂)_{P} mit _{P} = 1 bis 3 oder Alkenyl mit C₂ bis C₆ einsetzt.

4. Verfahren gemäß Anspruch 3, **dadurch gekennzeichnet, daß** m eine ganze Zahl von 0-4 ist.

5. Verfahren gemäß Anspruch 1, **dadurch gekennzeichnet, daß** man Hydroxysäuren der allgemeinen Formel
R₂-CO₂H (I)
einsetzt, in der bedeuten:
R₂: Aryl, HO-(CH₂)ₒ, oder CH₃(CHOH)ₙ(CH₂)ₘ- ist, wobei m eine ganze Zahl von 0 bis 20, n: eine ganze Zahl von 1 bis 5, und o: eine ganze Zahl von 1 bis 6 ist.

6. Verfahren gemäß Anspruch 5, **dadurch gekennzeichnet, daß** R₂ Phenylen oder Naphtyl, jeweils durch OH substituiert ist, m eine ganze Zahl von 0 bis 8 darstellt, und n eine ganze Zahl von 1 bis 4 ist.

7. Verfahren gemäß Anspruch 1, **dadurch gekennzeichnet, daß** man eine Hydroxygruppen-haltige Aminosäure einsetzt.

8. Verfahren gemäß Anspruch 7, **dadurch gekennzeichnet, daß** man Serin oder Threonin einsetzt.

9. Verfahren gemäß den Ansprüchen 1, 3 und 5, **dadurch gekennzeichnet, daß** man eine Mischung aus ortho-Borsäure und Oxalsäure oder Salicylsäure als Katalysator einsetzt.

10. Verfahren gemäß einem oder mehreren der vorhergehenden Ansprüche, **dadurch gekennzeichnet, daß** man eine Mischung aus ortho-Borsäure. einerseits und Oxalsäure, Salicylsäure, Weinsäure oder Zitronensäure andererseits in einem Molverhältnis von 1 : 1 bis 1 : 10, als Katalysator einsetzt.

11. Verfahren gemäß Anspruch 10, **dadurch gekennzeichnet, daß** das Molverhältnis der Mischung aus ortho-Borsäure einerseits und Oxalsäure, Salicylsäure, Weinsäure oder Zitronensäure andererseits von 1:2 bis 1:5 ist.

12. Verfahren gemäß einem oder mehreren der.vorhergehenden Ansprüche, **dadurch gekennzeichnet, daß** man eine Mischung aus dem Bor-haltigen Bestandteil und einer oder mehrerer der genannten Carbonsäuren in einer Menge von 0,1 bis 10 mol%, bezogen auf das eingesetzte Ketoisophoron, einsetzt.

13. Verfahren gemäß einem oder mehreren der vorhergehenden Ansprüche, **dadurch gekennzeichnet, daß** man ein Acylierungsmitel der allgemeinen Formel 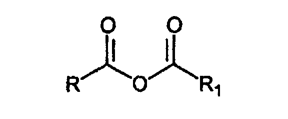 einsetzt, in der bedeuten:
R, R₁, gleich oder verschieden: einen, gegebenenfalls substituierten aliphatischen, alicyclischen C₁ bis C₂₀-Rest oder Aryl.

14. Verfahren gemäß Anspruch 13, **dadurch gekennzeichnet, daß** R und R₁ ein aliphatischer C₂ bis C₄-Rest oder Phenylen sind.

15. Verfahren gemäß einem oder mehreren der vorhergehenden Ansprüche, **dadurch gekennzeichnet, daß** man als Acylierungsmittel ein Carbonsäurechlorid einsetzt.

16. Verfahren gemäß Anspruch 1, **dadurch gekennzeichnet, daß** man den entstandenen Trimethylhydrochinondiester ohne vorherige Isolierung, gegebenenfalls nach destillativer Entfernung von nicht umgesetztem Acetanhydrid und entstandener Essigsäure, durch Zugabe von Wasser und/oder verdünnter Säure verseift und das entstandene TMHQ isoliert oder für Folgeumsetzungen ohne Isolierung weiter einsetzt.

17. Verfahren gemäß Anspruch 1, **dadurch gekennzeichnet, daß** man als Säure eine Verbindung der allgemeinen Formeln (I) und (II) einsetzt.

18. Verfahren gemäß einem oder mehreren der vorhergehenden Ansprüche, **dadurch gekennzeichnet, daß** man das Verfahren kontinuierlich durchführt, indem man die katalytisch wirksamen Säuren durch Extraktion mit einem polaren Lösungsmittel abtrennt und nach der Aufarbeitung wieder einsetzt.

## Claims

1. Process for the preparation of trimethylhydroquinone diesters 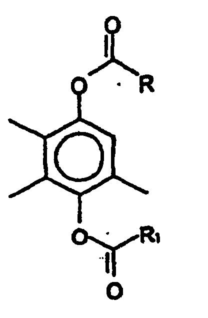 wherein R and R₁ are an optionally substituted aliphatic, alicyclic C₁ to C₂₀ radical, or are aryl, and wherein R and R₁ are identical or different, by reaction of 2,6,6-trimethyl-cyclohex-2-ene-1,4-dione (ketoisophorone or KIP) 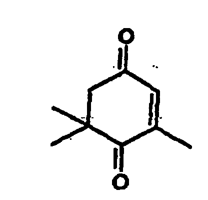 with an acylating agent in the presence of catalytic amounts of a protonic acid, **characterised in that** there are used as the acid catalyst ortho-boric acid and/or boric anhydride or a boric acid triester on the one hand and one or more carboxylic acid(s), selected from the group of the hydroxycarboxylic acids, the di- or tri-carboxylic acids, which optionally also contain hydroxy groups, on the other hand.

2. Process according to claim 1, **characterised in that** R and R₁ are an aliphatic C₂ to C₄ radical or are phenylene.

3. Process according to claim 1, **characterised in that** there are used dicarboxylic acids of the general formula
HO₂C-R₃-CO₂H (II)
in which R₃ is aryl or (CH₂)ₘ, wherein m represents an integer from 0 to 20, or (CH₂)ₘ(CHX)ₒ, wherein o corresponds to an integer from 1 to 5, where X: OH, H or (CH₂)ₚ-(CX) (COOH)-(CH₂)ₚ, wherein p = from 1 to 3, or C₂-C₆-alkenyl.

4. Process according to claim 3, **characterised in that** m is an integer from 0 to 4.

5. Process according to claim 1, **characterised in that** there are used hydroxy acids of the general formula
R₂-CO₂H (I)
in which:
R₂ is aryl, HO-(CH₂)ₒ or CH₃(CHOH)ₙ(CH₂)ₘ-, wherein m is an integer from 0 to 20, n is an integer from 1 to 5 and o is an integer from 1 to 6.

6. Process according to claim 5, **characterised in that** R₂ is phenylene or naphthyl, each substituted by OH, m represents an integer from 0 to 8 and n is an integer from 1 to 4.

7. Process according to claim 1, **characterised in that** an amino acid containing hydroxy groups is used.

8. Process according to claim 7, **characterised in that** serine or threonine is used.

9. Process according to claims 1, 3 and 5, **characterised in that** a mixture of ortho-boric acid and oxalic acid or salicylic acid is used as the catalyst.

10. Process according to one or more of the preceding claims, **characterised in that** there is used as the catalyst a mixture of ortho-boric acid on the one hand and oxalic acid, salicylic acid, tartaric acid or citric acid on the other hand in a molar ratio of from 1:1 to 1:10.

11. Process according to claim 10, **characterised in that** the molar ratio of the mixture of ortho-boric acid on the one hand and oxalic acid, salicylic acid, tartaric acid or citric acid on the other hand is from 1:2 to 1:5.

12. Process according to one or more of the preceding claims, **characterised in that** there is used a mixture of the boron-containing constituent and of one or more of the mentioned carboxylic acids in an amount of from 0.1 to 10 mol%, based on the ketoisophorone used.

13. Process according to one or more of the preceding claims, **characterised in that** there is used an acylating agent of the general formula 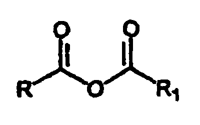 in which:
R, R₁ are identical or different and represent an optionally substituted aliphatic, alicyclic C₁ to C₂₀ radical, or aryl.

14. Process according to claim 13, **characterised in that** R and R₁ are an aliphatic C₂ to C₄ radical or are phenylene.

15. Process according to one or more of the preceding claims, **characterised in that** a carboxylic acid chloride is used as the acylating agent.

16. Process according to claim 1, **characterised in that** the resulting trimethylhydroquinone diester, without previously being isolated and optionally after removal by distillation of unreacted acetic anhydride and of acetic acid that has formed, is saponified by addition of water and/or dilute acid, and the resulting TMHQ is isolated or is used further for subsequent reactions without being isolated.

17. Process according to claim 1, **characterised in that** a compound of the general formulae (I) and (II) is used as the acid.

18. Process according to one or more of the preceding claims, **characterised in that** the process is carried out continuously by separating off the catalytically active acids by extraction with a polar solvent and using them again after working up.

## Revendications

1. Procédé pour la préparation de diesters de triméthylhydroquinone 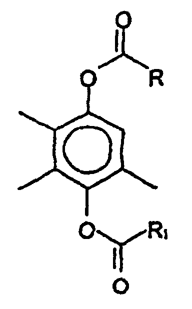 R et R₁ représentant un radical le cas échéant substitué, aliphatique, alicyclique en C₁ à C₂₀ ou aryle et R et R₁ étant identiques ou différents
par transformation de 2,6,6-triméthylcyclohex-2-ène-1,4-dione (cétoisophorone ou CIP) 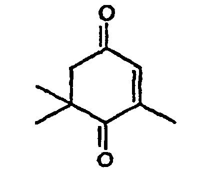 avec un agent d'acylation en présence de quantités catalytiques d'un acide protonique, **caractérisé en ce qu'**on utilise comme catalyseur acide de l'acide orthoborique et/ou de l'anhydride de l'acide borique ou un triester de l'acide borique d'une part et un ou plusieurs acides carboxyliques, choisis parmi le groupe des acides hydroxycarboxylique, dicarboxyliques ou tricarboxyliques, qui contiennent le cas échéant également des groupes hydroxy, d'autre part.

2. Procédé selon la revendication 1, **caractérisé en ce que** R et R₁ représentent un radical aliphatique en C₂ à C₄ ou phénylène.

3. Procédé selon la revendication 1, **caractérisé en ce qu'**on utilise des acides dicarboxyliques de formule générale
HO₂C-R₃-CO₂H (II)
dans laquelle R₃ représente un radical aryle ou un groupe (CH₂)ₘ, m représentant un nombre entier de 0 à 20, ou un groupe (CH₂)ₘ(CHX)ₒ, o correspondant à un nombre entier de 1 à 5, avec X = OH, H, ou un groupe (CH₂)ₚ-(CX)(COOH)-(CH₂)ₚ avec p = 1 à 3 ou un groupe alcényle en C₂ à C₆.

4. Procédé selon la revendication 3, **caractérisé en ce que** m est un nombre entier de 0 à 4.

5. Procédé selon la revendication 1, **caractérisé en ce qu'**on utilise des hydroxyacides de formule générale
R₂-CO₂H (I)
dans laquelle
R₂ signifie un groupe aryle, HO-(CH₂)ₒ ou CH₃(CHOH)ₙ(CH₂)ₘ-, m représentant un nombre entier de 0 à 20, n un nombre entier de 1 à 5 et o un nombre entier de 1 à 6.

6. Procédé selon la revendication 5, **caractérisé en ce que** R₂ représente un groupe phénylène ou un naphtyle, à chaque fois substitué par OH, m un nombre entier de 0 à 8 et n un nombre entier de 1 à 4.

7. Procédé selon la revendication 1, **caractérisé en ce qu'**on utilise un acide aminé contenant des groupes hydroxy.

8. Procédé selon la revendication 7, **caractérisé en ce qu'**on utilise de la sérine ou de la thréonine.

9. Procédé selon les revendications 1, 3 et 5, **caractérisé en ce qu'**on utilise comme catalyseur un mélange d'acide orthoborique et d'acide oxalique ou d'acide salicylique.

10. Procédé selon l'une ou plusieurs des revendications précédentes, **caractérisé en ce qu'**on utilise comme catalyseur un mélange d'acide orthoborique d'une part et d'acide oxalique, salicylique, tartrique ou citrique d'autre part, dans un rapport molaire de 1:1 à 1:10.

11. Procédé selon la revendication 10, **caractérisé en ce que** le rapport molaire du mélange d'acide orthoborique d'une part et d'acide oxalique, salicylique, tartrique ou citrique d'autre part est de 1:2 à 1:5.

12. Procédé selon l'une ou plusieurs des revendications précédentes, **caractérisé en ce qu'**on utilise un mélange d'un constituant contenant du bore et d'un ou plusieurs des acides carboxyliques mentionnés en une quantité de 0,1 à 10% en mole par rapport à la cétoisophorone utilisée.

13. Procédé selon l'une ou plusieurs des revendications précédentes, **caractérisé en ce qu'**on utilise un agent d'acylation de formule générale 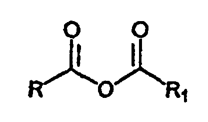 dans laquelle
R et R₁ sont identiques ou différents et représentent un radical le cas échéant substitué, aliphatique, alicyclique en C₁ à C₂₀ ou aryle.

14. Procédé selon la revendication 13, **caractérisé en ce que** R et R₁ représentent un radical aliphatique en C₂ à C₄ ou phénylène.

15. Procédé selon l'une ou plusieurs des revendications précédentes, **caractérisé en ce qu'**on utilise comme agent d'acylation un chlorure d'acide carboxylique.

16. Procédé selon la revendication 1, **caractérisé en ce qu'**on saponifie le diester de triméthylhydroquinone formé sans isolement préalable, le cas échéant après élimination par distillation de l'anhydride de l'acide acétique non transformé et de l'acide acétique formé, par addition d'eau et/ou d'acide dilué et qu'on isole la TMHQ formée ou qu'on l'utilise sans isolement pour des transformations consécutives.

17. Procédé selon la revendication 1, **caractérisé en ce qu'**on utilise comme acide un composé de formule générale (I) et (II).

18. Procédé selon l'une ou plusieurs des revendications précédentes, **caractérisé en ce qu'**on effectue le procédé en continu, **en ce qu'**on sépare les acides catalytiquement actifs par extraction avec un solvant polaire et qu'on les utilise à nouveau après le traitement.
